(19) Europäisches Patentamt
European Patent Office
Office européen des brevets

(11) Publication number: **0 279 937 B1**

(12) **EUROPEAN PATENT SPECIFICATION**

(45) Date of publication of patent specification: **09.10.91**

(51) Int. Cl.[5]: **C07C 237/06**, C07K 5/06, //A61K31/16,A61K37/02

(21) Application number: **87118506.2**

(22) Date of filing: **14.12.87**

# (54) 2-Aminoacetamide derivatives.

(30) Priority: **06.02.87 US 11982**

(43) Date of publication of application: **31.08.88 Bulletin 88/35**

(45) Publication of the grant of the patent: **09.10.91 Bulletin 91/41**

(84) Designated Contracting States: **AT BE CH DE ES FR GB IT LI LU NL SE**

(56) References cited:
**DE-C- 955 508**

**CHEMICAL ABSTRACTS, vol. 77, no. 3, 17th July 1972, page 506, abstract no. 19586g, Columbus, Ohio, US; P.B. PATEL et al.: "Potential local anesthetics. VII. Synthesis of diethylamino-, morpholino-, and piperidino-N-(substituted-1,2-diarylethyl)acetamides", & J. INDIAN CHEM. SOC. 1972, 49(2), 177-80**

(73) Proprietor: **FISONS CORPORATION
Jefferson Road
Rochester, NY 14603(US)**

(72) Inventor: **Griffith, Ronald C.
41 Northfield Gate
Pittsford New York 14534(US)**
Inventor: **Napier, James J.
34 Battlegreen Drive
Rochester New York 14624(US)**

(74) Representative: **Wright, Robert Gordon McRae
FISONS plc 12 Derby Road
Loughborough Leicestershire LE11 0BB(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

Novel substituted 2-aminoacetamide derivatives have been prepared and found to possess useful sedative and especially antiepileptic activity.

### General Description

This invention relates to novel 2-aminoacetamide compounds of the following general structure (1):

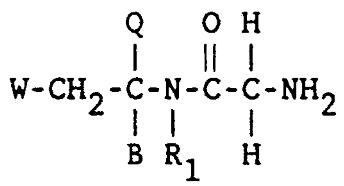

(1)

wherein B is hydrogen, lower alkyl ($C_1$-$C_4$) or methoxycarbonyl; $R_1$ is hydrogen or methyl and where W and Q are independently selected from phenyl or 4-fluorophenyl.

This invention also relates to optical isomers and to pharmaceutically acceptable acid addition salts of the compounds of general formula (1).

Compounds of this invention possess useful pharmaceutical properties. In particular they possess sedative and antiepileptic properties. Especially useful compounds are those in which B is hydrogen or methyl and W and Q are phenyl.

### Detailed Description

The 2-aminoacetamides of general formula (1) as described fully above are conveniently prepared by suitable amide bond forming reactions from the corresponding amine intermediates of general formula (2):

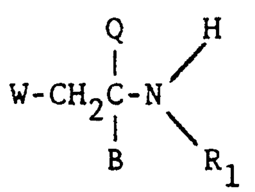

(2)

where B is hydrogen, lower alkyl ($C_1$-$C_4$), or methoxycarbonyl, $R_1$ is hydrogen or methyl, and where W and Q are independently selected from phenyl or 4-fluorophenyl and optical isomers thereof. Most of the amines of general formula (2) are known compounds and may be purchased commercially or conveniently prepared by suitable modifications of the reported procedures. Some of the amines (2) are not known, but are prepared by similar procedures. The preparation of the non-commercially available amines of general formula (2) is described in the "Preparation of Intermediates" Section.

Many amide bond forming reactions may in principle be utilized for the conversion of the amines of general formula (2) to the amides of general formula (1). Two procedures which represent the preferred methods for this conversion are designated Method A and Method B.

Method A consists of direct coupling of commercially available suitably protected aminoacid derivatives of formula (3):

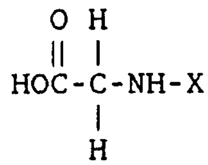

(3)

where X is an urethane protecting group preferably benzyloxycarbonyl (CBZ) or t-butyloxycarbonyl (BOC), with an amine of general formula (2), in an inert solvent in the presence of a coupling reagent such as dicyclohexylcarbodiimide with or without 1-hydroxybenzotriazole or other additives to provide coupled products of general formula (4):

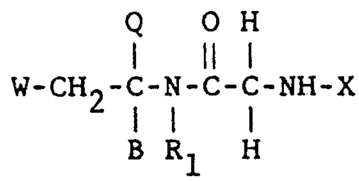

(4)

The protecting groups X, are then readily removed by either catalytic hydrogenation for the CBZ groups or treatment with an acid such as trifluoroacetic or hydrochloric acid for the BOC group to provide the compounds of general formula (1).

Method B consists of reacting an amine of general formula (2) with an activated two carbon acid derivative which contains a leaving group alpha to the carbonyl, such as chloroacetyl chloride, in the presence of an acid acceptor, such as triethylamine, to produce the corresponding 2-chloroacetamide derivative of general formula (5):

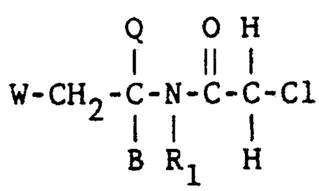

(5)

Such an intermediate can be directly reacted with ammonia in a solvent such as a lower alkanol, for example methanol or ethanol, or a chlorinated solvent, for example chloroform or methylene chloride or mixtures thereof to provide the corresponding compounds of general formula (1).

The compounds of general formula (1) possess an asymmetric center, and therefore optical isomers are possible. Such compounds are conveniently prepared from optically active amines of formula (2) by the methods described above.

The compounds of general formula (1) are basic compounds and may be used as such or pharmaceutically acceptable acid addition salts may be prepared by treatment with various inorganic or organic acids, such as hydrochloric, hydrobromic, sulfuric, phosphoric, acetic, lactic, succinic, fumaric, malic, maleic, tartaric, citric, benzoic, methanesulfonic or carbonic acids.

The compounds of general formula (1) possess useful pharmaceutical properties. In particular they possess useful antiepileptic properties and they possess sedative properties. These activities were assessed by standard methods. Antiepileptic activity was measured by assessing a compound's ability to prevent the hind limb tonic extension component of the seizure in groups of mice induced by maximal electroshock (MES) after oral or intraperitoneal administration according to the procedures of the Epilepsy Branch, NINCDS as published by R. J. Porter, et al., Cleve. Clin. Quarterly1984, 51, 293, and compared to the standard agents dilantin and phenobarbital. Activities ($ED_{50}$'s) in the range of 10-400 m/k after oral administration in this assay system were obtained. Sedative activity was assessed by behavioral observation in groups of mice. Selected compounds exhibited activity in the range of 30-600 m/k in this assay.

An important factor in judging the usefulness of antiepileptic agents is an evaluation of their propensity to produce neurotoxic effects (R. J. Porter, Cleve. Clin. Quarterly, 1984, 51, 293). Selected compounds were evaluated in an acute neurological impairment (NI) assay and $NI_{50}$ doses determined in mice essentially according to the procedure of Coughenour, et al., Pharmac. Biochem. Behav., 1977, 6, 351. The oral therapeutic index (TI), that is, the $NI_{50}$ in the neurological impairment assay divided by the $ED_{50}$ in the maximal electroshock assay after oral doses, was calculated. Unusually high oral therapeutic indices were observed.

The following non-limiting illustrations and examples are provided to exemplify the preparation of the intermediate amines of formula (2) and their conversion to the novel compounds of general formula (1).

## Preparation of Intermediates

### Illustration 1

### Preparation of 1,2-Diphenyl-2-propylamine hydrochloride

This compound was prepared by suitable modification of the procedures described by Christol, Bull Soc. Chim. Fr., 1963, 4 877, and Ho and Smith, Tetrahedron, 1970, 26, 4277 as follows. To a suspension of sodium cyanide (34.3 g, 0.7 mol) in 500 ml of glacial acetic acid and 100 ml of n-butylether at 0° C was added portionwise 200 ml of concentrated sulfuric acid. The ice bath was removed and a solution of 1,2-diphenyl-2-propanol (106 g, 0.5 ml) in 100 ml of n-butylether was added dropwise over a period of 2 hours, then the mixture stirred for 48 hours. The mixture was poured into 1000 cc of ice, and extracted with chloroform. The extracts were washed with water, dried and evaporated to a solid residue which was stirred with hexane (500 ml), filtered and dried to give 85.35 g (72% yield) of N-formyl-1,2-diphenyl-2-propylamine, mp 97-99° C. This was suspended in 1 L of 10% HCl and heated to reflux for 2.5 hours. After cooling in air for 1 hour then in an ice bath for 30 minutes, the white solid which had crystallized was collected by filtration and vacuum dried to give 85.9 g (97% yield) of 1,2-diphenyl-2-propylamine hydrochloride, mp 175-178° C.

### Illustration 2

### Preparation of 1,2-bis(4-fluorophenyl)-2-propylamine hydrochloride

By procedures essentially the same as those described in Illustration 1, and by substituting 1,2-bis(4-fluorophenyl)-2-propanol (prepared by the reaction of 4-fluorobenzyl magnesium chloride and 4'-fluoroacetophenone) for 1,2-diphenyl-2-propanol; the corresponding 1,2-bis(4-fluorophenyl-2-propylamine hydrochloride, mp 188-189° C, is prepared.

### Illustration 3

### Preparation of 1,2-Diphenyl-2-butylamine hydrochloride

By procedures essentially the same as those described in Illustration 1, and by substituting 1,2-diphenyl-2-butanol (prepared by the reaction of benzylmagnesium chloride and propiophenone) for 1,2-diphenyl-2-propanol; the corresponding 1,2-diphenyl-2-butylamine hydrochloride, mp 190-192.5° C, is prepared.

### Illustration 4

### Preparation of (-)-1,2-diphenyl-2-propylamine

Racemic 1,2-diphenyl-2-propylamine (86 g, 0.4 mol) was dissolved in 0.5 L 95% ethanol, heated to near reflux and added to a solution of (-)-dibenzoyltartaric acid monohydrate (151.9 g, 0.4 mol) in 0.5 L 95% ethanol also at reflux. A white solid crystallized immediately. The mixture was refluxed for 5 minutes, then allowed to cool to ambient temperature. The solid was collected by filtration and dried to give 86.2 g ($[\alpha]_D$ = -94.2°; C = 0.5, $CH_3OH$). The filtrate was saved. The solid was suspended in 0.9 L of 95% ethanol, stirred and heated to reflux for 1 hour, allowed to cool to ambient temperature and the white solid collected by filtration and vacuum dried at 80 °C for 8 hours to give 60.2 g of (-)-1,2-diphenyl-2-propylamine-(-)-dibenzoyl tartrate, mp 194-195 °C; $[\alpha]_D$ = -96.0° (C = 0.5, $CH_3OH$). 5.0 g of this salt was dissolved in 250 ml $CHCl_3$ and 200 ml 5% $NH_4OH$ shaken vigorously, the layers separated and the organic phase washed with 3 x 200 ml 5% $NH_4OH$, 2 x 200 ml $H_2O$ and dried over $MgSO_4$. The solvent was evaporated to give 1.75 g of (-)-1,2-diphenyl-2-propylamine as an oil. The maleate salt was prepared by dissolving this oil in 25 ml of ethylacetate and adding the solution to a hot solution of maleic acid (1.02 g, 8.87 mmol) in 50 ml of 3/1 ethyl acetate/isopropanol. Upon cooling a white solid crystallized, which was collected by filtration and vacuum dried to give 2.05 g of (-)-1,2-diphenyl-2-propylamine maleate, mp 176-177 °C, $[\alpha]_D$ = -27.4°, (C = 1, $CH_3OH$).

Illustration 5

Preparation of (+)-1,2-Diphenyl-2-propylamine

The filtrate residue which was saved in Illustration 4, was treated with 1 L $CHCl_3$ and 0.9 L 5% $NH_4OH$, shaken vigorously, the layers separated and the organic phase washed with 4 x 800 ml 5% $NH_4OH$ and 2 x 500 ml $H_2O$, then dried over $MgSO_4$ and evaporated to an oil 32.3 g, which is enriched in (+)-1,2-diphenyl-2-propylamine. This oil (32.3 g, 0.153 mol) was dissolved in 200 ml hot 95% ethanol and added to a stirred solution of (+)-dibenzoyl tartaric acid monohydrate (57.55 g, 0.153 mol) in 600 ml of refluxing 95% ethanol. A white solid crystallized immediately, which was stirred at reflux for 5 minutes, then allowed to cool to ambient temperature. The solid was collected by filtration and vacuum dried at 80° for 8 hours to give 71.6 g of (+)-1,2-diphenyl-2-propylamine. (+)-dibenzoyltartrate, mp 197-198 °C, $[\alpha]_D$ = +95.8°, (C = 0.5, $CH_3OH$). 5.0 g of this salt was dissolved in 250 ml $CHCl_3$ and 200 ml 5% $NH_4OH$, shaken vigorously, the layers separated and the organic phase washed with 3 x 200 ml 5% $NH_4OH$ and 2 x 200 ml $H_2O$ and dried over $MgSO_4$. The solvent was evaporated to give 1.75 g of (+)-1,2-diphenyl-2-propylamine as an oil. The maleate salt was prepared by dissolving this oil in 25 ml ethyl acetate and adding the solution to a hot solution of maleic acid (1.02 g, 8.78 mmole) in 50 ml 3/1 ethylacetate/isopropanol. Upon cooling a white solid crystallized, which was collected by filtration and vacuum dried to give 2.06 g of (+)-1,2-diphenyl-2-propylamine maleate, mp 177-178 °C, $[\alpha]_D$ = +27.3° (C = 1, $CH_3OH$).

Illustration 6

Preparation of 2,3-Diphenyl-2-aminopropanoic acid methyl ester

To a suspension of NaH (21.0 g, 0.525 mol) (60% oil dispersion) in 400 ml of THF at 0 °C under nitrogen, was added dropwise a solution of N-[(4-chlorophenyl)methylene]-2-phenylglycine methyl ester (R. Grigg, Chem. Comm., 1980, 648) (143.9 g, 0.5 mol) in 500 ml DMF and 100 ml THF over a period of 2.5 hours. This was stirred at ca 10 °C for 1 hour, then a solution of benzyl bromide (61 ml, 0.5 mol) in 70 ml of THF was added and the mixture stirred at ambient temperature for 17 hours. The mixture was poured into 2 L of water, extracted with ethyl acetate (3 x 600 ml), the extracts washed with water (3 x 500 ml) dried and evaporated to an oil. This was dissolved in 400 ml of THF, 400 ml of methanol and 800 ml of 1N HCl and stirred for 1.5 hours, then extracted with 800 ml of ether, and 2 x 500 ml of ethyl acetate/ether (1/4). The combined organic extracts were washed with 500 ml 1N HCl, and the combined aqueous layers were basified to pH 10 with solid sodium carbonate, and extracted with chloroform (3 x 500 ml). The combined extracts were washed with water, dried and evaporated to a yellow solid, 121 g. This was recrystallized from hexane (400 ml) to give 76.3 g of 2,3-diphenyl-2-aminopropanoic acid methyl ester, mp 61-62 °C.

Illustration 7

Preparation of N-Methyl-1,2-diphenyl-2-propylamine hydrochloride

N-formyl-1,2-diphenyl-2-propylamine (23.6 g, 0.1 mol) was added to a stirred suspension of $LiAlH_4$ (15.0

g, 0.395 mol) in 1 L of dry tetrahydrofuran. After 2 hours the mixture was heated at 35° C for 22 hours, then refluxed for 2 hours, and allowed to cool to room temperature. Water was added to decompose the excess $LiAlH_4$, and the mixture filtered to remove the solid salts. Evaporation of the solvent gave 23.0 g of the crude product as a yellow oil. This was dissolved in 180 ml of ethyl acetate and 20 ml of isopropanol and acidified with HCl gas. Upon standing a white solid crystallized which was collected by filtration and vacuum dried at 65° C to give 21.7 g (84%) of N-methyl-1,2-diphenyl-2-propylamine hydrochloride; mp 200-201° C.

Illustration 8

Preparation of N-Methyl-1,2-diphenylethylamine

To a stirred two phase solution of 1,2-diphenylethylamine (30.0 g, 0.15 mol) in 300 ml of methylene chloride and 500 ml of water was added sodium carbonate (23.9 g, 0.225 mol) and the solution was cooled to 10° C under nitrogen. Ethyl chloroformate (21.5 ml, 0.225 mol) was added dropwise over a one hour period. The reaction was warmed to ambient temperature and stirred at that temperature for 3 hours. The phases were separated and the aqueous phase was extracted with methylene chloride (75 ml). The combined methylene chloride extracts were washed with 1N HCl (200 ml), brine (200 ml), dried and evaporated to a white solid, 40.3 g. Recrystallization from cyclohexane gave N-carboethoxy-1,2-diphenylethylamine, mp 74-75° C.

To a stirred suspension of lithium aluminum hydride (12.4 g, 0.032 mol) in 300 ml of tetrahydrofuran at 0° C under nitrogen was added dropwise a solution of N-carboethoxy-1,2-diphenylethylamine (35.0 g, 0.13 mol) in 200 ml of tetrahydrofuran. The mixture was heated to reflux for 8 hours. The mixture was cooled in an ice-water bath and water (13 ml), 15% NaOH (13 ml) and water (39 ml) were carefully added to the mixture. The mixture was warmed to ambient temperature and the precipitated salts were removed by filtration through celite. Removal of solvent gave N-methyl-1,2-diphenylethylamine, 26.8 g as a colorless oil.

Treatment of this oil with maleic acid in ethyl acetate and methanol gave N-methyl-1,2-diphenylethylamine maleate, mp 129-131° C.

Example 1

Preparation of 2-Amino-N-(1,2-diphenyl-1-methylethyl)-acetamide hydrochloride

To a stirred solution of 1,2-diphenyl-2-propylamine (21.0 g, 0.085 mol) in 500 ml of chloroform under nitrogen was added N-CBZ-glycine (23.0 g, 0.11 mol), and then a solution of dicyclohexylcarbodiimide (20.6 g, 0.1 mol) in 100 ml of chloroform and the mixture stirred for 14 hours. The precipitated solid was removed by filtration and the solvent evaporated. The residue was dissolved in 500 ml of methylene chloride, filtered and evaporated to a yellow oil. This was treated with ether (750 ml) and 500 ml of ice cold water, basified with 5 ml of 50% NaOH, the layers shaken and separated. The ether layer was washed with water (2 x 125 ml), dried and evaporated to an oil, 33.5 g. This was dissolved in 500 ml of methanol and 50 ml of 10% HCl, and hydrogenated at 40 psi in a Parr apparatus over 3.0 g of 10% Pd/C catalyst for 4 hours. The catalyst was removed by filtration, and the solvent evaporated to a white solid. This was dissolved in 80 ml of hot methanol and treated with 200 ml of ether. Upon cooling a solid crystalized which was recrystallized from 100 ml of isopropanol and 100 ml of methanol to give 8.4 g of 2-amino-N-(1,2-diphenyl-1-methylethyl)-acetamide hydrochloride, which after vacuum drying at 80° C for 24 hours had mp 253-254° C.

Example 2

Preparation of 2-Amino-N-[1,2-bis(4-fluorophenyl)-1-methylethyl]acetamide

To a stirred solution of 1,2-bis(4-fluorophenyl)-2-propylamine (12.0 g, 0.049 mol) in 200 ml of chloroform under nitrogen, was added N-CBZ-glycine (10.16 g, 0.049 mol) and then a solution of dicyclohexylcarbodiimide (11.35 g, 0.055) in 100 ml of chloroform and the mixture stirred for 30 minutes, then filtered and the solvent evaporated. The residue was treated with ethyl acetate (200 ml), filtered, an additional 200 ml of ethyl acetate added, and then washed with 1% cold HCl (200 ml), brine (200 ml), dried, and the solvent evaporated to a pale yellow oil. This was dissolved in 400 ml of methanol and 35 ml of 10% HCl and hydrogenated at 40 psi in a Parr apparatus over 2.5 g of 5% Pd/C catalyst for 2.5 hours. The catalyst was removed by filtration, solvent evaporated and the residue dissolved in water (300 ml) and chloroform (500 ml), basified to pH 11 with 50% NaOH, shaken, and separated. The aqueous phase was extracted with

chlroform(2 x 200 ml) and the combined organic phases washed with water (2 x 150 ml), brine (150 ml), dried and evaporated to a pale yellow oil which solidified on standing. The solid was recrystallized three times from cyclohexane (150 ml) and ethanol (10 ml) and vacuum dried to give 4.44 g of 2-amino-N-[1,2-bis(4-fluorophenyl)-1-methylethyl]acetamide, mp 130-131 °C.

Example 3

Preparation of 2-Amino-N-(1-ethyl-1,2-diphenylethyl)acetamide

By procedures essentially the same as those described in Example 1 and substituting 1,2-diphenyl-2-butylamine for 1,2-diphenyl-2-propylamine; the corresponding 2-amino-N-(1-ethyl-1,2-diphenyelthyl)-acetamide, mp 108.5-109.5 °C, is prepared.

Example 4

Preparation of 2-Amino-N-(1,2-diphenylethyl)acetamide hydrochloride

By procedures essentially the same as those described in Example 1 and by substituting 1,2-diphenylethylamine for 1,2-diphenyl-2-propylamine, the corresponding 2-amino-N-(1,2-diphenylethyl)-acetamide hydrochloride, mp 197-199 °C, is prepared.

Example 5

Preparation of 2-Amino-N-[1,2-diphenyl-1-(methoxycarbonyl)ethyl]acetamide maleate

To a stirred solution of 2,3-diphenyl-2-aminopropanoic acid methyl ester, (7.0 g, 0.0275 mol) in 210 ml of chloroform under nitrogen was added N-CBZ-glycine (6.31 g, 0.028 mol) and then dropwise a solution of dicyclohexylcarbodiimide (6.22 g, 0.028 mol) in 85 ml of chloroform, and the mixture stirred for 20 hours. Precipitated solids were removed by filtration, and the solvent evaporated to an oily residue which was dissolved in 200 ml of ethyl acetate, filtered, washed with 200 ml of 1N HCl, 200 ml of 1N sodium carbonate, and 200 ml of brine, then dried and evaporated to an oily residue, 14.29 g. 12.5 g of this material was dissolved in 210 ml of methanol and 60 ml of 1N HCl and hydrogenated at 40 psi in a Parr apparatus over 1.0 g of 10% Pd/C catalyst for 3 hours. The catalyst was removed by filtration and the solvent evaporated to a semisolid residue. This was dissolved in water (100 ml) and ethyl acetate (100 ml) and basified with 1N sodium carbonate. The layers were separated, the aqueous layer extracted with ethyl acetate (2 x 100 ml) and the combined organic layers washed with brine, and dried. This solution was treated with 3.25 g of maleic acid and evaporated to an off-white solid, 9.89 g. This was recrystallized from 100 ml of 1:1 methanol:ethyl acetate to give after drying 4.58 g of 2-amino-N-[1,2-diphenyl-1-(methoxycarbonyl)ethyl]acetamide maleate as a white solid, mp 163-165 °C.

Example 6

Preparation of (+)-2-Amino-N-(1,2-diphenyl-1-methylethyl)acetamide fumarate

By procedures essentially the same as those described in Example 1, and by substituting (-)-1,2-diphenyl-2-propylamine for (±)-1,2-diphenyl-2-propylamine; the corresponding (+)-2-amino-N-(1,2-diphenyl-1-methylethyl)acetamide fumarate, mp 169-170 °C, $[\alpha]_D$ = 10.3° (C = 2, $CH_3OH$), is prepared.

Example 7

Preparation of (-)-2-Amino-N-(1,2-diphenyl-1-methylethyl)acetamide fumarate

By procedures essentially the same as those described in Example 1, and by substituting (+)-1,2-diphenyl-2-propylamine for (±)-1,2-diphenyl-2-propylamine; the corresponding (-)-2-amino-N-(1,2-diphenyl-1-methylethyl)acetamide fumarate, mp 171-172 °C, $[\alpha]_D$ = -9.4° (C = 2, $CH_3OH$), is prepared.

Example 8

Preparation of 2-Amino-N-methyl-N-(1,2-diphenylethyl)acetamide maleate

To a stirred solution of N-methyl-1,2-diphenylethylamine (25.95 g, 0.123 mol) and triethylamine (44.5 ml, 0.32 mol) in 300 ml of methylene chloride at 4°C under nitrogen, was added dropwise a solution of chloroacetyl chloride (12.9 ml, 0.16 mol) in 50 ml of methylene chloride. The ice bath was removed and the mixture stirred overnight. Water (300 ml) was added and the layers separated. The aqueous layer was extracted with methylene chloride (100 ml). The combined organic layers were washed with 1N HCl (200 ml), brine (100 ml), dried and evaporated to a dark oil, 40.6 g. This oil was treated with hot hexane (4 x 100 ml) and then cyclohexane (100 ml). The combined hexane solutions were allowed to cool to ambient temperature. An off white solid crystallized, and was isolated by filtration to give 14.2 g of the chloroacetamide, mp 90-92°C. Recrystallization from isopropanol gave material of mp 96-97°C. The above chloroacetamide (10.0 g, 0.034 mol) was suspended in 200 ml of ammonia saturated ethanol, and the mixture heated to 85-90°C for 20 hours in a steel bomb. The mixture was cooled to room temperature and the solvent evaporated. The residue was dissolved in 5% NaOH (100 ml) and chloroform (300 ml), the layers separated, and the aqueous layer extracted with chloroform (2 x 50 ml). The combined chloroform extracts were washed with brine (100 ml) and chloroform (300 ml), the layers separated, and the aqueous layer extracted with chloroform (2 x 50 ml). The combined chloroform extracts were washed with brine (100 ml), dried and evaporated to a dark oil, 13.1 g. This oil was purified by chromatography on a Prep 500 HPLC on silica gel eluting with 5% methanol/chloroform. Pure fractions were combined and evaporated to give 5.9 g of an oil. This was dissolved in ethyl acetate (100 ml) and methanol (25 ml) and treated with maleic acid (2.55 g) and carbon, hot filtered, concentrated to a volume of 60 ml and diluted to 100 ml with ethyl acetate. Upon cooling a white solid crystallized, which was vacuum dried to give 5.76 g of 2-amino-N-methyl-N-(1,2-diphenylethyl)acetamide maleate, mp 150-151°C.

Example 9

Preparation of 2-Amino-N-methyl-N-(1,2-diphenyl-1-methylethyl)acetamide maleate

By procedures essentially the same as those described in Example 8 and by substituting N-methyl-1,2-diphenyl-2-propylamine for N-methyl-1,2-diphenylethylamine; the corresponding 2-chloro-N-methyl-N-(1,2-diphenyl-1-methylethyl)acetamide, mp 109-110°C, and 2-amino-N-methyl-N-(1,2-diphenyl-1-methyl-ethyl)-acetamide maleate, mp 166-168°C are prepared.

Comparative Results

Oral therapeutic indices (TI) were calculated for the compounds of Examples 1 and 4 from the test procedures previously described. TI's of 17.0 and 20.0 respectively were obtained. The compounds 2-(dimethylamino)-N-(1,2-diphenylethyl)acetamide and 2-(1-pyrrolidinyl)-N-(1,2-diphenylethyl)acetamide (disclosed in German patent 955,508, dated January 3, 1957) were prepared and tested by the same procedures. TI's of 8.8 and 2.2 respectively were obtained.

**Claims**

**Claims for the following Contracting States : AT, BE, CH, DE, FR, GB, IT, LI, LU, NL, SE**

**1.** A compound of the formula

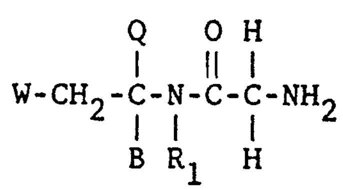

wherein B is hydrogen, lower alkyl ($C_1$-$C_4$) or methoxycarbonyl; $R_1$ is hydrogen or methyl and where W and Q are independently selected from phenyl or 4-fluorophenyl.

**2.** 2-Amino-N-(1,2-diphenyl-1-methylethyl)acetamide hydrochloride.

**3.** 2-Amino-N-[1,2-bis(4-fluorophenyl)-1-methylethyl] acetamide.

**4.** 2-Amino-N-(1-ethyl-1,2-diphenylethyl)acetamide.

**5.** 2-Amino-N-(1,2-diphenylethyl)acetamide hydrochloride.

**6.** 2-Amino-N-[1,2-diphenyl-1-(methoxycarbonyl)ethyl]-acetamide maleate.

**7.** (+)-2-Amino-N-(1,2-diphenyl-1-methy1ethyl)acetamide fumarate.

**8.** (-)-2-Amino-N-(1,2-diphenyl-1-methylethyl)acetamide fumarate.

**9.** 2-Amino-N-methyl-N-(1,2-diphenylethyl)acetamide maleate.

**10.** 2-Amino-N-methyl-N-(1,2-diphenyl-1-methylethyl) acetamide maleate.

**Claims for the following Contracting State : ES**

**1.** A process for preparing a compound of the formula:

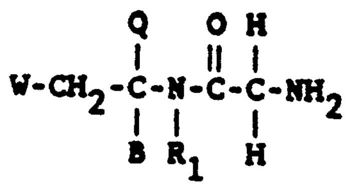

(1)

wherein B is hydrogen, lower alkyl ($C_1$-$C_4$) or methoxycarbonyl; $R_1$ is hydrogen or methyl and where W and Q are independently selected from phenyl or 4-fluorophenyl which comprises reacting an amine of the formula

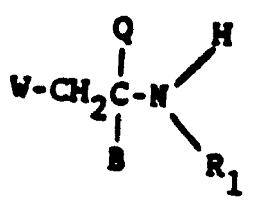

with a suitably protected amino acid of the formula

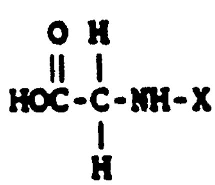

where X is a urethane protecting group and thereafter removing the protecting group by catalytic

hydrogenation or treatment with an acid.

**2.** A process for preparing a compound according to claim 1 wherein the compound is 2-amino-N-(1,2-diphenyl-1-methylethyl)acetamide hydrochloride.

**3.** A process for preparing a compound according to claim 1 wherein the compound is 2-amino-N-[1,2-bis-(4-fluorophenyl)-1-methylethyl]acetamide.

**4.** A process for preparing a compound according to claim 1 wherein the compound is 2-amino-N-(1-ethyl-1,2-diphenylethyl)acetamide.

**5.** A process for preparing a compound according to claim 1 wherein the compound is 2-amino-N-(1,2-diphenylethyl) acetamide hydrochloride.

**6.** A process for preparing a compound according to claim 1 wherein the compound is 2-amino-N-[1,2-diphenyl-1-(methyoxycarbonyl)ethyl]acetamide maleate.

**7.** A process for preparing a compound according to claim 1 wherein the compound is (+)-2-amino-N-(1,2-diphenyl-1-methylethyl)acetamide fumarate.

**8.** A process for preparing a compound according to claim 1 wherein the compound is (-)-2-amino-N-(1,2-diphenyl-1-methylethyl)acetamide fumarate.

**9.** A process for preparing a compound according to claim 1 wherein the compound is 2-amino-N-methy1-N-(1,2-diphenylethyl) acetamide maleate.

**10.** A process for preparing a compound according to claim 1 wherein the compound is 2-amino-N-methyl-N-(1,2-diphenyl-1-methylethyl)acetamide maleate.

**Revendications**

**Revendications pour les Etats contractants suivants : AT, BE, CH, DE, FR, GB, IT, LI, LU, NL, SE**

**1.** Composé de formule

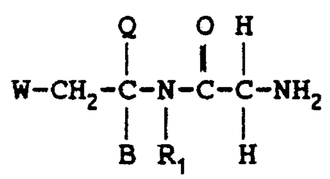

où B est hydrogène, alcoyle inférieur ($C_1$-$C_4$) ou méthoxycarbonyle,
$R_1$ est hydrogène ou méthyle, et
W et Q sont choisis indépendamment entre phényle et 4-fluorophényle.

**2.** Le chlorhydrate de 2-amino-N-(1,2-diphényl-1-méthyléthyl) acétamide.

**3.** Le 2-amino-N-[1,2-bis(4-fluorophényl)-1-méthyléthyl) acétamide.

**4.** Le 2-amino-N-(1-éthyl-1,2-diphényl-éthyl)-acétamide.

**5.** Le chlorhydrate de 2-amino-N-(1,2-diphényl-éthyl)acétamide.

**6.** Le maléate de 2-amino-N-[1,2-diphényl-1-(méthoxycarbonyl) éthyl] acétamide.

**7.** Le fumarate de (+) -2-amino-N-(1, 2-diphényl-1-méthyléthyl) acétamide.

8. Le fumarate de (-)-2-amino-N-(1,2-diphényl-1-méthyléthyl) acétamide.

9. Le maléate de 2-amino-N-méthyl-N-(1,2-diphényléthyl) acétamide.

10. Le maléate de 2-amino-N-méthyl-N-(1,2-diphényl-1-méthyléthyl) acétamide.

**Revendications pour l'Etat contractant suivant : ES**

1. Procédé de préparation d'un composé de la formule :

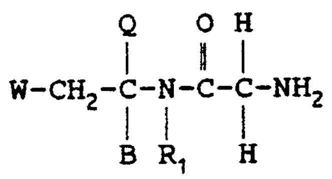

(1)

où B est hydrogène, alcoyle inférieur ($C_1$-$C_4$) ou méthoxycarbonyle,
$R_1$ est hydrogène ou méthyle, et
W et Q sont choisis indépendamment entre phényle et 4-fluorophényle,
qui comprend la réaction d'une amine de la formule

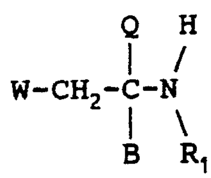

avec un acide aminé convenablement protégé de la formule

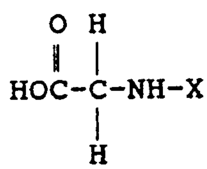

où X est un radioal protecteur de la fonction uréthanne, puis l'élimination du radical protecteur par hydrogénation catalytique ou par traitement avec un acide.

2. Procédé de préparation d'un composé suivant la revendication 1, dans lequel le composé est le chlorhydrate de 2-amino-N-(1,2-diphényl-1-méthyléthyl)-acétamide.

3. Procédé de préparation d'un composé suivant la revendication 1, dans lequel le composé est le 2-amino-N-[1,2-bis(4-fluorophényl)-1-méthyléthyl]acétamide.

4. Procédé de préparation d'un composé suivant la revendication 1, dans lequel le composé est le 2-amino-N-(1-éthyl-1,2-diphényléthyl)acétamide.

5. Procédé de préparation d'un composé suivant la revendication 1, dans lequel le composé est le chlorhydrate de 2-amino-N-(1,2-diphényléthyl)acétamide.

6. Procédé de préparation d'un composé suivant la revendication 1, dans lequel le composé est le

maléate de 2-amino-N-[1,2-diphényl-1-(méthoxycarbonyl)-éthyl]-acétamide.

7. Procédé de préparation d'un composé suivant la revendication 1, dans lequel le composé est le fumarate de (+)-2-amino-N-(1,2-diphényl-1-méthyléthyl)acétamide.

8. Procédé de préparation d'un composé suivant la revendication 1, dans lequel le composé est le fumarate de (-)-2-amino-N-(1,2-diphényl-1-méthyléthyl)acétamide.

9. Procédé de préparation d'un composé suivant la revendication 1, dans lequel le composé est le maléate de 2-amino-N-méthyl-N-(1,2-diphényléthyl)acétamide.

10. Procédé de préparation d'un composé suivant la revendication 1, dans lequel le composé est le maléate de 2-amino-N-méthyl-N-(1,2-diphényl-1-méthyléthyl)acétamide.

**Patentansprüche**
**Patentansprüche für folgende Vertragsstaaten : AT, BE, CH, DE, FR, GB, IT, LI, LU, NL, SE**

1. Verbindung der Formel:

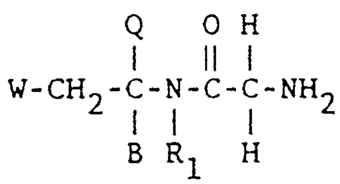

worin B Wasserstoff, Niedrigalkyl-($C_1$-$C_4$) oder Methoxycarbo-nyl bedeutet; $R_1$ Wasserstoff oder Methyl bedeutet und worin W und Q unabhängig ausgewählt werden unter Phenyl oder 4-Fluorphenyl.

2. 2-Amino-N-(1,2-diphenyl-1-methylethyl)-acetamid-hydrochlorid.

3. 2-Amino-N-[1,2-bis-(4-fluorphenyl)-1-methylethyl]-acetamid.

4. 2-Amino-N-(1-ethyl-1,2-diphenylethyl)-acetamid.

5. 2-Amino-N-(1,2-diphenylethyl)-acetamid-hydrochlorid.

6. 2-Amino-N-[1,2-diphenyl-1-(methoxycarbonyl)-ethyl]-acetamid-maleat.

7. (+)-2-Amino-N-(1,2-diphenyl-1-methylethyl)-acetamid-fumarat.

8. (-)-2-Amino-N-(1,2-diphenyl-1-methylethyl)-acetamid-fumarat.

9. 2-Amino-N-methyl-N-(1,2-diphenylethyl)-acetamid-maleat.

10. 2-Amino-N-methyl-N-(1,2-diphenyl-1-methylethyl)-acetamid-maleat.

**Patentansprüche für folgenden Vertragsstaat : ES**

1. Verfahren zur Herstellung einer Verbindung der Formel:

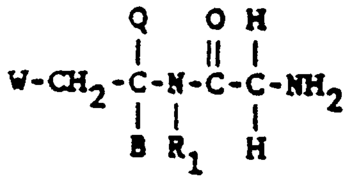

(1)

worin B Wasserstoff, Niedrigalkyl-($C_1$-$C_4$) oder Methoxycarbonyl bedeutet; $R_1$ Wasserstoff oder Methyl bedeutet und worin W und Q unabhängig ausgewählt werden unter Phenyl oder 4-Fluorphenyl, dadurch **gekennzeichnet,** daß ein Amin der Formel:

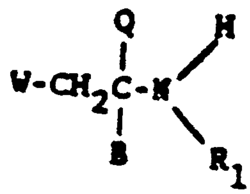

mit einer geeigneten geschützten Aminosäure der Formel:

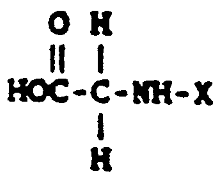

worin X eine Urethan-Schutzgruppe bedeutet, umgesetzt wird und anschließend die Schutzgruppe durch katalytische Hydrierung oder Behandlung mit einer Säure entfernt wird.

2. Verfahren zur Herstellung einer Verbindung nach Anspruch 1, dadurch **gekennzeichnet,** daß die Verbindung 2-Amino-N-(1,2-diphenyl-1-methylethyl)-acetamid-hydrochlorid ist.

3. Verfahren zur Herstellung einer Verbindung nach Anspruch 1, dadurch **gekennzeichnet,** daß die Verbindung 2-Amino-N-[1,2-bis-(4-fluorphenyl)-1-methyl-ethyl]-acetamid ist.

4. Verfahren zur Herstellung einer Verbindung nach Anspruch 1, dadurch **gekennzeichnet,** daß die Verbindung 2-Amino-N-(1-ethyl-1,2-diphenylethyl)-acetamid ist.

5. Verfahren zur Herstellung einer Verbindung nach Anspruch 1, dadurch **gekennzeichnet,** daß die Verbindung 2-Amino-N-(1,2-diphenylethyl)-acetamid-hydrochlorid ist.

6. Verfahren zur Herstellung einer Verbindung nach Anspruch 1, dadurch **gekennzeichnet,** daß die Verbindung 2-Amino-N-[1,2-diphenyl-1-(methoxycarbonyl)-ethyl]-acetamid-maleat ist.

7. Verfahren zur Herstellung einer Verbindung nach Anspruch 1, dadurch **gekennzeichnet,** daß die Verbindung (+)-2-Amino-N-(1,2-diphenyl-1-methylethyl)-acetamid-fumarat ist.

8. Verfahren zur Herstellung einer Verbindung nach Anspruch 1, dadurch **gekennzeichnet,** daß die Verbindung (-)-2-Amino-N-(1,2-diphenyl-1-methylethyl)-acetamid-fumarat ist.

9. Verfahren zur Herstellung einer Verbindung nach Anspruch 1, dadurch **gekennzeichnet,** daß die Verbindung 2-Amino-N-methyl-N-(1,2-diphenylethyl)-acetamid-maleat ist.

10. Verfahren zur Herstellung einer Verbindung nach Anspruch 1, dadurch **gekennzeichnet,** daß die Verbindung 2-Amino-N-methyl-N-(1,2-diphenyl-1-methylethyl)-acetamid-maleat ist.